# EUROPÄISCHE PATENTANMELDUNG

(11) **EP 1 752 438 A2**
(43) Veröffentlichungstag der Anmeldung: **14.02.2007**
(21) Anmeldenummer: 06014869.9
(22) Anmeldetag: 18.07.2006
(51) Int. Cl.: C07C 45/63, C07C 49/80

(54) **Verfahren zur Herstellung von gegebenenfalls substituiertem Trifluormethylphenacylbromid**

(30) Priorität: 28.07.2005 DE 102005035301
(71) Anmelder: Saltigo GmbH, 51369 Leverkusen (DE)
(72) Erfinder: Berkenbusch, Thilo, 50668 Köln (DE); Schlummer, Björn, 53115 Bonn (DE)
(74) Vertreter: Pettrich, Klaus-Günter

(57) **Zusammenfassung**

Die vorliegende Erfindung betrifft ein Verfahren zur Herstellung von gegebenenfalls substituiertem Trifluormethylphenacylbromid, insbesondere von 3-Trifluormethylphenacylbromid der Formel (I-a)

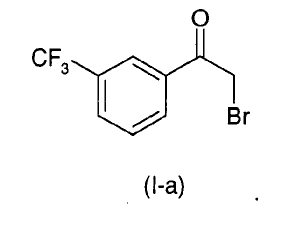

## Beschreibung

Die vorliegende Erfindung betrifft ein Verfahren zur Herstellung von gegebenenfalls substituiertem Trifluormethylphenacylbromid, insbesondere von 3-Trifluormethylphenacylbromid.

Die Darstellung von 3-Trifluormethylphenacylbromid mittels Bromierung von 3-Trifluormethylacetophenon ist bekannt. So ist beispielsweise die Verwendung von elementarem Brom in Chloroform *(*J. Heterocyclic. Chem. 1981, 18, 451-453.) oder Diethylether (J. Med. Chem. 1995, 38, 34-41.), elementarem Brom mit Aluminiumtrichlorid in Diethylether (J. prakt. Chem. 1998, 340, 649-655.) oder Pyridiniumtribromid in bromwasserstoffhaltiger Essigäure (Chem. Pharm. Bull. 1995, 43, 1497-1504.) als Bromierungsreagenzien beschrieben. Die in der Literatur beschriebenen Ausbeuten liegen dabei in der Regel bei 70 bis 90%.

Allerdings entsteht als zwangsläufiges Nebenprodukt der obigen Umsetzung unabhängig von der Bromierungsmethode das Dibromderivat α,α-Dibrom-3-trifluormethylacetophenon. Sofern in der Folgeumsetzung nicht das Rohprodukt eingesetzt werden kann und soll, wird als Aufarbeitungs- bzw. Reinigungsmethode in der Literatur vorzugsweise ein destillatives Verfahren beschrieben. Eine Aufreinigung des Rohproduktes mittels Destillation bringt jedoch den Nachteil mit sich, dass sich 3-Trifluormethylphenacylbromid unter thermischer Belastung bei Temperaturen oberhalb von 100°C im Rahmen einer Gleichgewichtsreaktion zersetzt und ein destillatives Verfahren unter Verwendung erhöhter Temperaturen zur Isolation von 3-Trifluormethylphenacylbromid untauglich ist. Als Zersetzungsprodukte entstehen 3-Trifluormethylacetophenon, das Edukt der Bromierungsreaktion, und α,α-Dibrom-3-trifluormethylacetophenon. Dabei liegt die Gleichgewichtseinstellung - ausgehend von theoretisch reinem 3-Trifluormethylphenacylbromid - bei ca. 10:80:10 für 3-Trifluormethylacetophenon : 3-Trifluormethyl-phenacylbromid : α,α-Dibrom-3-trifluormethylacetophenon. Der maximal erreichbare Gehalt an mittels Destillation gereinigtem liegt aufgrund dessen bei ca. 80 Gewichts-%. In der Literatur sind keinerlei Angaben hinsichtlich der Reinheit der Rohprodukte oder der Destillate zu finden.

3-Trifluormethylphenacylbromid stellt einen wertvollen Baustein für eine kettenverlängernde Ketonsynthese dar und kann darüber hinaus für den Aufbau von Heterozyklen wie z. B. Arylimidazole *(*Bioorganic & Medicinal Letters 2003, 13, 3593-3596) oder Arylthiophenderivate *(*Bioorganic & Medicinal Letters 2002, 10, 1953-1957) genutzt werden. Daher ist es wünschenswert, Material von hoher Reinheit herzustellen, da andernfalls ein Verlust des unter Umständen wertvollen anderen Kupplungsbausteins durch Umsetzung mit dem α,α-Dibrom-3-trifluormethylacetophenon sowie eine anschließende Trennung der Kupplungsprodukte in Kauf genommen werden muss.

In J. Am. Chem. Soc. 1961, 83, 4277-4281 wird die Bromierung von 3-Trifluormethylacetophenon mit elementarem Brom in Eisessig beschrieben, gefolgt von anschließender Entfernung gebildeter HBr mittels Eintragen in Eiswasser und Ausetherung, Entfernen des Ethers und Reinigung des Rohproduktes mittels Kristallisation in Ethanol. Nachteilig bei diesem Verfahren ist die gute Mischbarkeit des Solvens Eisessig mit Wasser, welche die Notwendigkeit der für eine großtechnische Anwendung ungünstigen zusätzlichen Ausetherung zur Trennung des gewünschten Produktes von der hoch korrosiven HBr bedingt. Durch die aufwändige Aufarbeitung ist mit einer verringerten Ausbeute an gewünschtem Produkt zu rechnen. Allerdings sind auch in J. Am. Chem. Soc. 1961, 83, 4277-4281 keinerlei Angaben hinsichtlich Ausbeuten oder Reinheiten sowohl des Rohproduktes als auch des 3-Trifluormethylphenacylbromids zu finden.

Es bestand somit weiterhin Bedarf an einem effizienten Verfahren zur Herstellung von Trifluormethylphenacylbromiden, insbesondere 3-Trifluormethylphenacylbromid, bei dem das gewünschte Produkt in hoher Reinheit erhalten wird.

Die Aufgabe der vorliegenden Erfindung lag somit darin ein solches Verfahren bereitzustellen. Ein weiterer Aspekt der vorliegenden Erfindung lag darin, dem Verlust von Edukt durch etwaige Nebenreaktionen zu minimieren.

Überraschend wurde gefunden, dass ein Verfahren zur Herstellung von gegebenenfalls substituierten Trifluormethylphenacylbromiden, insbesondere 3-Trifluormethylphenacylbromid, mittels Bromierung von gegebenenfalls substituiertem Trifluormethylacetophenon, insbesondere 3-Trifluormethylacetophenon, zu Produkten in hoher Reinheit führt, wenn das erhaltene Rohprodukt im Anschluss an die Bromierung aus einem geeigneten organischen Lösungsmittel bei Temperaturen von -78°C bis +15°C kristallisiert wird.

Gegenstand der vorliegenden Erfindung ist somit ein Verfahren zur Herstellung von gegebenenfalls durch einen oder mehrere weitere(n) gleiche oder unterschiedliche Rest(e) am Aromaten substituiertem Trifluormethylphenacylbromid der Formel (I), 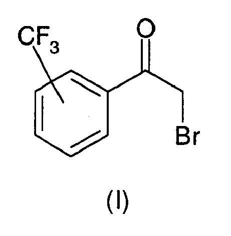 wobei gegebenenfalls substituiertes Trifluormethylacetophenon der Formel (II), 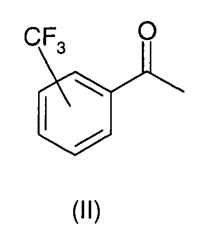 mit wenigstens einem Bromierungsreagenz bromiert wird, dadurch gekennzeichnet, dass das gegebenenfalls substituierte Trifluormethylphenacylbromid der Formel (I) aus dem nach der Bromierung erhaltenen Rohprodukt in wenigstens einem organischen Lösungsmittel bei wenigstens einer Temperatur von -78°C bis +15°C auskristallisiert wird.

Die CF₃-Gruppe in den Verbindungen der Formel (I) oder (II) kann sich in 2-, 3- oder 4-Position des Aromaten bezogen auf die Position der Acyl- bzw. Acylbromidgruppe befinden. Bevorzugt ist sie in 3-Position.

Der aromatische Ring kann gegebenenfalls weiter durch einen oder mehrere gleiche oder verschiedene Reste substituiert sein, die unabhängig voneinander gegebenenfalls substituiertes lineares, verzweigtes oder cylisches C₁-C₁₈-Alkyl, bevorzugt C₁-C₆-Alkyl, C₁-C₁₈-Alkoxy, bevorzugt C₁-C₆-Alkoxy, C₁-C₁₈-Halogenalkyl, bevorzugt C₁-C₆-Halogenalkyl, C₁-C₁₈-Aminoalkyl, bevorzugt C₁-C₆-Aminoalkyl, C₄-C₂₄-Aryl, bevorzugt C₆-C₂₄-Aryl, C₄-C₂₄-Aryloxy, bevorzugt C₆-C₂₄-Aryloxy, C₅-C₁₈-Arylalkyl, Halogen, Nitro, Hydroxy oder Carboxyl.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Insbesondere eignet sich das erfindungsgemäße Verfahren zur Herstellung von 3-Trifluormethylphenacylbromid der Formel (I-a)

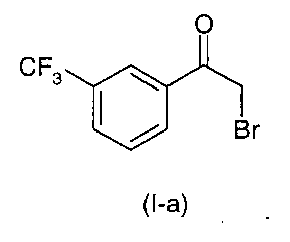

Die Bromierung kann in Abwesenheit eines Lösungsmittels oder in Gegenwart wenigstens eines geeigneten Lösungsmittels erfolgen. Als geeignete Lösungsmittel für die Bromierung kommen insbesondere mit Wasser schlecht bis nicht mischbare Lösungsmittel oder Lösungsmittelmischungen enthaltend wenigstens eines dieser Lösungsmittel in Frage. Solche schlecht bis nicht mit Wasser mischbaren Lösungsmittel sind im Rahmen der Erfindung solche, von denen sich weniger als 10 Gew.-% bezogen auf die Gesamtmasse des Wassers mit Wassers mischen lassen.

Solche mit Wasser schlecht bis nicht mischbaren Lösungsmittel sind beispielsweise aliphatische oder aromatische Kohlenwasserstoffe, wie z.B. n-Hexan, n-Pentan, Cyclohexan, Benzol, Toluol und Xylol, chlorierte aliphatische oder aromatische Kohlenwasserstoffe, wie z.B. Dichlormethan, Chloroform, 1,2-Dichlorethan, Chlorbenzol, Chortoluol und Dichlortoluol, oder aliphatische oder cycloaliphatische Ether, wie z.B. Methyl-*tert*-Butylether (MTBE), Diethylether, Tetrahydrofuran (THF), 2-Methyltetrahydrofuran (Methyl-THF) oder Dioxan.

In besonderen Ausführungsformen der Erfindung können aber auch mit Wasser mischbare organische Lösungsmittel bei der Bromierung verwendet werden. Hierbei eignen sich besonders organische Carbonsäuren, wie z.B. Ameisensäure, Essigsäure und Propionsäure.

Die Bromierung kann mit geeigneten, dem Fachmann bekannten Bromierungsreagenzien durchgeführt werden. Beispielsweise geeignet sind elementares Brom, Pyridiniumtribromid, N-Bromsuccinimid oder 1,3-Dibrom-5,5-dimethylhydantoin gegebenenfalls in Gegenwart einer Lewis-Säure und/oder Brönsted-Säure und gegebenenfalls in einem geeigneten Lösungsmittel. Als Lösungsmittel eignen sich vorzugsweise auch die für die Bromierung als Lösungsmittel verwendeten, es können für die Bromierungsreagenzien jedoch auch andere Lösungsmittel eingesetzt werden. Als Lewis-Säuren kommen beispielsweise Aluminiumtrichlorid, Aluminiumtribromid, Zink(II)chlorid, Zink(II)bromid, Galliumtribromid, Galliumtrichlorid oder Bortrifluoridetherat (BF₃·OEt₂) in Frage, als Brönsted-Säuren HBr, H₂SO₄, H₃PO₄, HCl, HF, organische Carbonsäuren, wie z.B. Ameisensäure, Essigsäure, Trifluoressigsäure und Propionsäure oder Sulfonsäuren, wie z.B. Methansulfonsäure, *para*-Toluolsulfonsäure oder Trifluormethansulfonsäure.

Bevorzugt werden für die Kristallisation als organisches Lösungsmittel aliphatische oder cycloaliphatische Ether, wie z.B. Methyl-*tert*-Butylether (MTBE), Diethylether, Tetrahydrofuran (THF), 2-Methyltetrahydrofuran (Methyl-THF) oder Dioxan, aliphatische oder cycloaliphatische Kohlenwasserstoffe, z.B. n-Hexan, n-Pentan oder Cyclohexan, halogenierte aliphatische und aromatische Kohlenwasserstoffe, wie z.B. Dichlormethan, Chloroform, 1,2-Dichlorethan, Chlorbenzol, Dichlorbenzol oder Chlortoluol, aromatische Kohlenwasserstoffe, wie z.B. Benzol, Toluol oder Xylol, Alkohole, wie z.B. Methanol, Ethanol, 1,2-Ethandiol (Glykol), n-Propanol, 2-Propanol, n-Butanol, sec-Butanol, sec- oder tert-Amylalkohol, oder Mischungen enthaltend ein oder mehrere dieser Lösungsmittel eingesetzt. Besonders bevorzugte organische Lösungsmittel sind Alkohole, insbesondere Methanol, Ethanol, n-Propanol, 2-Propanol, n-Butanol, sec-Butanol, sec- oder tert-Amylalkohol, halogenierte aliphatische Kohlenwasserstoffe, insbesondere Dichlormethan, Chloroform oder 1,2-Dichlorethan, oder aliphatische Kohlenwasserstoffe, insbesondere n-Hexan oder n-Pentan. Ganz besonders bevorzugt sind Ethanol, 2-Propanol oder n-Hexan oder Mischungen enthaltend ein oder mehrere dieser Lösungsmittel.

Weiterhin bevorzugt wird die Kristallisation des gegebenenfalls substituierten Trifluormethylphenacylbromids der Formel (I) bei wenigstens einer Temperatur von -30°C bis 0°C, bevorzugt von -15°C bis 5°C durchgeführt.

Das organische Lösungsmittel und das Rohprodukt werden bei der Kristallisation vorzugsweise in einem Volumenverhältnis von 6: 1 bis 1:6, besonders bevorzugt von 1:1 bis 1:5, ganz besonders bevorzugt von1:2,5 bis 1:3,5 eingesetzt.

Die Kristallisation kann bei normalem, erhöhtem oder vermindertem Druck durchgeführt werden, beispielsweise im Bereich von 0,5 bis 50 bar. Bevorzugt wird die Kristallisation unter Siedekühlung, d.h. bei einem Druck kleiner als 1 bar durchgeführt.

In einer bevorzugten Ausführungsform des erfindungsgemäßen Verfahrens kann die bei der Kristallisation erhaltene Mutterlauge wieder in den Prozess der Bromierung zurückgeführt werden, was den Vorteil aufweist, dass gegebenenfalls nicht umgesetztes Edukt der Formel (II) wieder in den Prozess zurückgeführt wird und Verluste minimiert werden.

Bei dieser bevorzugten Ausführungsform des erfindungsgemäßen Verfahrens kann die Bromierung auch in Gegenwart eines anderen als einem mit Wasser schlecht oder nicht mischbaren Lösungsmittel erfolgen. Solche geeigneten Lösungsmittel können beispielsweise mit Wasser mischbare organische Lösungsmittel sein. Hierbei eignen sich besonders organische Carbonsäuren, wie z.B. Ameisensäure, Essigsäure - gegebenenfalls in Form von Eisessig - und Propionsäure.

In einer besonders bevorzugten Ausführungsform kann die Mutterlauge vor der Zurückführung in den Bromierungsprozess einer thermischen Isomerisierung unterzogen werden. Diese thermische Isomerisierung, vorzugsweise bei wenigstens einer Temperatur von 80°C oder darüber, besonders bevorzugt von 100°C oder darüber, ganz besonders bevorzugt von 100°C bis 160°C, bedingt die Einstellung eines temperaturabhängigen Gleichgewichts (in Gleichung (1) beispielhaft für 3-Trifluormethylphenacylbromid (I-a) gezeigt), 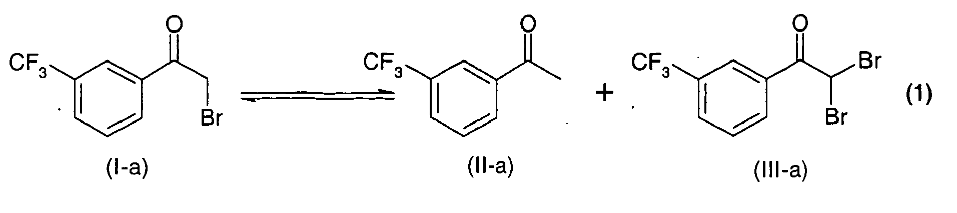 worin die gegebenenfalls substituierten Trifluormethylphenacylbromide der Formel (I) im Überschuss vorliegen. Diese Isomerisierung hat den Vorteil, dass nicht umgesetztes Edukt der Formel (II) mit den dibromierten Nebenprodukten (in Gleichung (1) der Formel (III-a)) zu einem großen Anteil in gewünschtes Produkt überführt werden kann und durch Rückführung in den Prozess nicht verloren geht. Auf diese Weise können insbesondere Verluste durch das unbrauchbare dibromierte Nebenprodukt deutlich reduziert werden.

Die thermische Isomerisierung kann über einen Zeitraum von wenigen Minuten bis mehreren Stunden durchgeführt werden, vorzugsweise wird sie für einen Zeitraum von 30 min bis 20 h durchgeführt. Die Durchführung der thermischen Isomerisierung unter Schutzgasatmosphäre, wie z.B. Stickstoff- oder Argonatmosphäre, kann von Vorteil sein, ist jedoch nicht zwingend erforderlich. Die thermische Isomerisierung kann bei normalem, erhöhtem oder vermindertem Druck durchgeführt werden, beispielsweise im Bereich von 0,5 bis 50 bar. Im Allgemeinen wird sie bei Normaldruck durchgeführt.

Mit dem erfindungsgemäßen Verfahren können die gewünschten Trifluormethylphenacylbromide der Formel (I) in einer Reinheit von mehr als 90 Gew.-%, bevorzugt sogar in einer Reinheit von mehr als 95 Gew.-% erhalten werden. Zersetzung des gewünschten Produktes durch thermische Isomerisierung bei Destillation kann mit dem erfindungsgemäßen Verfahren vermieden werden.

Die folgenden Beispiele dienen der beispielhaften Veranschaulichung der Erfindung und sind nicht als Beschränkung aufzufassen.

### Beispiele

### Beispiel 1:

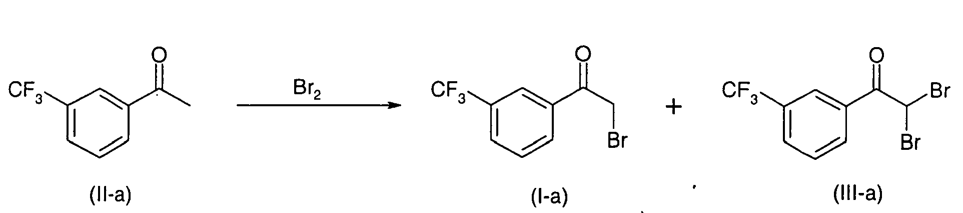

### 1.1. Darstellung von 3-Trifluormethylacetophenon (I-a)

Eine Lösung von 3-Trifluormethylacetophenon ((II-a), 300 g, 1.59 mol) in Methylenchlorid (900 mL) wurde bei Raumtemperatur innerhalb von 4 h tropfenweise mit Brom (255 g, 1.60 mol) versetzt. Man ließ 15 h rühren, versetzte mit wässriger gesättigter Natriumhydrogencarbonat-Lösung (600 mL) und trennte die Phasen. Die organische Phase wurde erneut mit wässriger gesättigter Natriumhydrogencarbonat-Lösung (600 mL) gewaschen, über Magnesiumsulfat getrocknet und unter vermindertem Druck vom Solvens befreit. Man erhielt das Rohprodukt (424 g), das laut ¹H-NMR-Analyse 76.2 Gew.-% 3-Trifluormethylphenacylbromid ((I-a), 75.9% Ausbeute) sowie 4.6 Gew.-% 3-Trifluormethylacetophenon (II-a) und 12.5 Gew.-% α,α-Dibrom-3-trifluormethylacetophenon (III-a) enthielt.

### 1.2.a) Kristallisation von 3-Trifluormethylacetophenon (I-a) aus Ethanol

Eine Lösung von 3-Trifluormethylphenacylbromid **((I-a),** 150 g, 76.2%-ig, 0.428 mol) enthaltend 4.6 Gew.-% Trifluormethylacetophenon **(II-a)** und 12.5 Gew.-% α,α-Dibrom-3-trifluormethylacetophenon **(III-a))** wurde mit Ethanol (40 mL) versetzt und 10 h bei -10°C stehen gelassen. Der entstandene Niederschlag wurde mit n-Hexan (2 x 10 mL) gewaschen und bei -10°C und 20 mbar getrocknet. Man erhielt 3-Trifluormethylphenacylbromid **((I-a),** 50.0 g, 41.9%) in einer Reinheit von 95.8 Gew.-%, welches zu 1.7 Gew.-% 3-Trifluormethylacetophenon **(II-a)** und 2.6 Gew.-% α,α-Dibrom-3-trifluormethylacetophenon **(III-a)** enthielt.

### 1.2.b) Kristallisation von 3-Trifluormethylacetophenon (I-a) aus Ethanol, 2-Propanol und n-Hexan

Eine Lösung von 3-Trifluormethylphenacylbromid **((I-a),** 30.0 g, 81.7%-ig, 91.7 mmol) enthaltend zu 7.0 Gew.-% 3-Trifluormethylacetophenon **(II-a)** und 7.6 Gew.-% α,α-Dibrom-3-trifluormethylacetophenon **(III-a))** wurde mit dem jeweils in Tab. 1 angegeben Lösungsmittel (10 mL) versetzt, auf -10°C gekühlt und nach Animpfen 6 h stehen gelassen. Die ausgefallenen Kristalle wurden abfiltriert, mit wenig n-Hexan gewaschen und bei -10°C und 20 mbar getrocknet. Die Auswaagen (nach Trocknung), Ausbeuten und Reinheiten sind jeweils in Tab. 1 angegeben.

**Tab. 1**

| **Lösungsmittel** | **Auswaage [g]** | **Reinheit [%]** | **Ausbeute [%]** |
|---|---|---|---|
| EtOH | 8.2 | 97.3 | 32.6 |
| 2-Propanol | 8.1 | 96.4 | 31.8 |
| n-Hexan | 7.2 | 96.3 | 28.3 |

### Beispiel 2: Untersuchung des thermischen Gleichgewichts von 3-Trifluormethylacetophenon (II-a), 3-Trifluormethylphenacylbromid (I-a) und α,α-Dibrom-3-trifluormethylacetophenon (III-a)

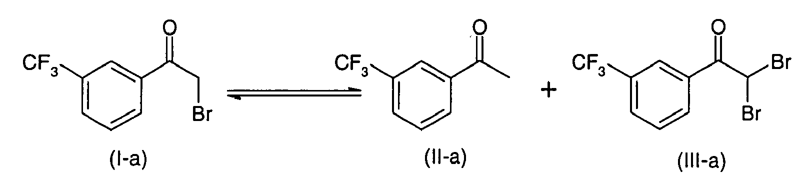

### 2.1. Kurzzeituntersuchung bei 100°C, 130°C und 150°C:

3-Trifluormethylphenacylbromid **(I-a,** 2.0 g, 96.9 GC-Flächen-%, 7.3 mmol; enthält zu 1.2 GC-Flächen-% 3-Trifluormethylacetophenon **(II-a)** und 1.5 GC-Flächen-% α,α-Dibrom-3-trifluormethylacetophenon **(III-a))** in einem Reagenzglas mit Schraubkappe und Thermometer wurde in ein 100°C oder 130°C oder 150°C temperiertes Ölbad (Tab. 2-4) gehalten. Nach den in den Tab. 2-4 angegeben Minutenabständen wurde eine Probe entnommen und per GC analysiert. Die Gleichgewichtszusammensetzungen sind in Tab. 2-4 angegeben.

**Tab. 2: Temperatur des Ölbads = 100°C; *Angaben in GC-Flächen-%**

| **Zeit [min]** | **T [°C]** | **Molverhältnisse [%]*** | | |
|---|---|---|---|---|
| | (Reagenzglas) | **II-a** | **I-a** | **III-a** |
| 0 | 25 | 1.2 | 96.9 | 1.5 |
| 1 | 78 | 1.3 | 96.8 | 1.6 |
| 3 | 94 | 1.4 | 96.3 | 1.7 |
| 5 | 97 | 1.5 | 96.4 | 1.8 |
| 8 | 97 | 1.5 | 96.2 | 1.9 |
| 11 | 98 | 1.6 | 95.9 | 2.0 |

**Tab. 3: Temperatur des Ölbads = 130°C; *Angaben in GC-Flächen-%**

| **Zeit [min]** | **T [°C]** | **Molverhältnisse [%]*** | | |
|---|---|---|---|---|
| | (Reagenzglas) | **II-a** | **I-a** | **III-a** |
| 0 | 25 | 1.2 | 96.9 | 1.5 |
| 1 | 95 | 1.3 | 96.5 | 1.7 |
| 3 | 124 | 1.7 | 96.1 | 2.0 |
| 5 | 127 | 1.9 | 95.5 | 2.2 |
| 8 | 128 | 2.2 | 94.9 | 2.5 |
| 11 | 128 | 2.5 | 94.4 | 2.7 |

**Tab. 4: Temperatur des Ölbads = 150°C; *Angaben in GC-Flächen-%**

| **Zeit [min]** | **T [°C]** | **Molverhältnisse [%]*** | | |
|---|---|---|---|---|
| | (Reagenzglas) | **II-a** | **I-a** | **III-a** |
| 0 | 25 | 1.2 | 96.9 | 1.5 |
| 1 | 110 | 1.5 | 96.3 | 1.8 |
| 3 | 143 | 2.1 | 95.0 | 2.4 |
| 5 | 147 | 2.5 | 94.3 | 2.7 |
| 8 | 148 | 3.3 | 93.1 | 3.4 |
| 11 | 148 | 3.8 | 91.9 | 4.0 |

### 2.2. Langzeituntersuchung bei 150°C:

a) 3-Trifluormethylphenacylbromid (**I-a,** 2.0 g, 96.7 GC-Flächen-%, 7.5 mmol; enthält zu 1.1 GC-Flächen-% 3-Trifluormethylacetophenon (**II-a**) und 1.6 GC-Flächen-% α,α-Dibrom-3-trifluormethylacetophenon (**III-a**)) wurde unter Argon bei 150°C gerührt. In Abständen von jeweils 1.5 h wurde eine Probe entnommen und per GC analysiert. Die Gleichgewichtszusammensetzungen sind in Tab. 5 angegeben.

**Tab. 5**

| **Zeit [h]** | **Molverhältnisse [%]*** | | |
|---|---|---|---|
| | **II-a** | **I-a** | **III-a** |
| 0 | 1.1 | 96.7 | 1.6 |
| 1.5 | 6.9 | 83.7 | 9.1 |
| 3.0 | 8.1 | 79.1 | 12.4 |
| 4.5 | 8.2 | 77.1 | 14.2 |
| 6.0 | 7.5 | 76.1 | 15.7 |
| 7.5 | 8.0 | 74.9 | 16.4 |

| | | | |
|---|---|---|---|
| *Angaben in GC-Flächen-% | | | |

Sowohl die Kurzzeit- als auch die Langzeituntersuchungen des thermischen Gleichgewichts zeigen eindeutig die Zersetzung des gewünschten Produktes 3-Trifluormethylphenacylbromid bei erhöhten Temperaturen, d.h. unter Destillationsbedingungen. Die Beispiele 2.1 und 2.2 belegen somit, dass eine Reinigung des 3-Trifluormethylphenacylbromids mittels Destillation mit weiterem Ausbeuteverlust verbunden und daher von erheblichem Nachteil ist.

## Patentansprüche

1. Verfahren zur Herstellung von gegebenenfalls durch einen oder mehrere weitere(n) gleiche oder unterschiedliche Rest(e) am Aromaten substituiertem Trifluormethylphenacylbromid der Formel (I), 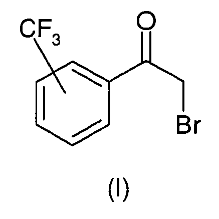 **dadurch gekennzeichnet, dass** gegebenenfalls substituiertes Trifluormethylacetophenon der Formel (II), 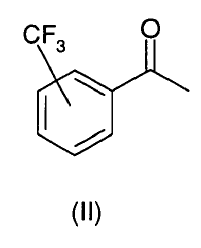 mit wenigstens einem Bromierungsreagenz in Abwesenheit eines Lösungsmittels oder in Gegenwart eines mit Wasser schlecht bis nicht mischbaren Lösungsmittels bromiert wird und das gegebenenfalls substituierte Trifluormethylphenacylbromid der Formel (I) aus dem nach der Bromierung erhaltenen Rohprodukt in wenigstens einem organischen Lösungsmittel bei wenigstens einer Temperatur von -78°C bis +15°C auskristallisiert wird.

2. Verfahren gemäß Anspruch 1, **dadurch gekennzeichnet, dass** als organisches Lösungsmittel aliphatische oder cycloaliphatische Ether, aliphatische oder cycloaliphatische Kohlenwasserstoffe, halogenierte aliphatische Kohlenwasserstoffe, aromatische Kohlenwasserstoffe, Alkohole oder Mischungen enthaltend ein oder mehrere dieser Lösungsmittel eingesetzt werden.

3. Verfahren gemäß Anspruch 1 oder 2, **dadurch gekennzeichnet, dass** als organisches Lösungsmittel Alkohole, bevorzugt Methanol, Ethanol, n-Propanol, 2-Propanol, n-Butanol, sec-Butanol, sec- oder tert-Amylalkohol, oder aliphatische Kohlenwasserstoffe, bevorzugt n-Hexan oder n-Pentan, eingesetzt werden.

4. Verfahren gemäß wenigstens einem der Ansprüche 1 bis 3, **dadurch gekennzeichnet, dass** als mit Wasser schlecht bis nicht mischbares Lösungsmittel aliphatische oder aromatische Kohlenwasserstoffe, chlorierte aliphatische oder aromatische Kohlenwasserstoffe oder aliphatische oder cycloaliphatische Ether eingesetzt werden.

5. Verfahren gemäß wenigstens einem der Ansprüche 1 bis 4, **dadurch gekennzeichnet, dass** das gegebenenfalls substituierte Trifluormethylphenacylbromid der Formel (I) bei wenigstens einer Temperatur von -30°C bis 0°C, bevorzugt von -15°C bis 5°C, auskristallisiert wird.

6. Verfahren gemäß wenigstens einem der Ansprüche 1 bis 5, **dadurch gekennzeichnet, dass** bei der Kristallisation das organische Lösungsmittel und das Rohprodukt in einem Volumenverhältnis von 6: 1 bis 1:6 eingesetzt werden.

7. Verfahren gemäß wenigstens einem der Ansprüche 1 bis 6, **dadurch gekennzeichnet, dass** als Bromierungsreagenz elementares Brom, Pyridiniumtribromid, N-Bromsuccinimid oder 1,3-Dibrom-5,5-dimethylhydantoin gegebenenfalls in Gegenwart einer Lewis-Säure und/oder Brönsted-Säure und gegebenenfalls in einem geeigneten Lösungsmittel eingesetzt werden.

8. Verfahren gemäß wenigstens einem der Ansprüche 1 bis 7, **dadurch gekennzeichnet, dass** die bei der Kristallisation erhaltene Mutterlauge wieder in den Prozess der Bromierung zurückgeführt wird.

9. Verfahren gemäß Anspruch 8, **dadurch gekennzeichnet, dass** die Mutterlauge vor der Zurückführung in den Bromierungsprozess einer thermischen Isomerisierung unterzogen wird.

10. Verfahren gemäß wenigstens einem der Ansprüche 1 bis 9, **dadurch gekennzeichnet, dass** die Kristallisation bei einem Druck kleiner als 1 bar durchgeführt wird.

11. Verfahren gemäß wenigstens einem der Ansprüche 1 bis 10, **dadurch gekennzeichnet, dass** gegebenenfalls substituiertes Trifluormethylphenacylbromid der Formel (I) in einer Reinheit von mindestens 95 Gew.-% erhalten wird.

12. Verfahren gemäß wenigstens einem der Ansprüche 1 bis 11, **dadurch gekennzeichnet, dass** als die Verbindung der Formel (I) 3-Trifluormethylphenacylbromid der Formel (I-a) 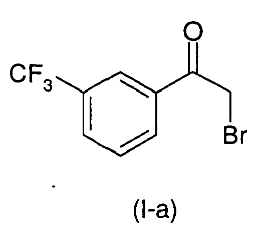 hergestellt wird.
